# FASCICULE DE BREVET EUROPEEN

(11) **EP 1 343 533 B1**
(45) Date de publication et mention de la délivrance du brevet: **11.08.2004**
(21) Numéro de dépôt: 01271915.9
(22) Date de dépôt: 12.12.2001
(51) Int. Cl.: A61K 51/04, A61K 51/12

(54) **DISPOSITIF DE SYNTHESE DE PRODUITS RADIOPHARMACEUTIQUES**
VORRICHTUNG ZUR SYNTHESE VON RADIOPHARMACA
DEVICE FOR SYNTHESIS OF RADIOPHARMACEUTICAL PRODUCTS

(30) Priorité: 22.12.2000 EP 00870323
(43) Date de publication de la demande: 17.09.2003
(73) Titulaire: ION BEAM APPLICATIONS S.A., 1348 Louvain-la-Neuve (BE)
(72) Inventeur: SCHMITZ, Frédéric, B-4260 FALLAIS (BE); BOEYEN, Pierre-Emmanuel, B-5032 CORROY-LE-CHATEAU (BE); TADINO, Vincent, B-4357 DONCEEL (BE)
(74) Mandataire: Van Malderen, Joelle
(86) Numéro de dépôt international: PCT/BE2001/000212
(87) Numéro de publication internationale: WO 2002/051447

(56) Documents cités:
- WO-A-93/11871
- US-A- 5 312 592
- US-A- 5 415 843
- US-A- 5 759 513
- CHALY, THOMAS ET AL: "A large scale manual production of [ fluorine - 18 ]FDG using a synthetic unit made of sterile disposable components an operated by a master slave manipulator" APPL. RADIAT. ISOT. (1990), 41(1), 29-34, XP001002876

## Description

### Objet de l'invention

La présente invention se rapporte à un nouveau dispositif pour la synthèse de produits radiopharmaceutiques à partir de composés radioactifs.

La présente invention se rapporte plus particulièrement à l'utilisation de ce dispositif pour synthèse de produits radiopharmaceutiques à partir de réactifs contenant des éléments radioactifs de courte demi-vie, tels que le ¹¹C, le ¹³N, le ¹⁵O ou le ¹⁸F.

### Etat de la technique

Dans des applications de diagnostic, telles que la tomographie par émission de positrons (Positron Emission Tomography-PET), on utilise des composés radiochimiques, encore appelés composés radiopharmaceutiques, qui sont marqués au moyen d'un élément tel que ¹¹C, ¹³N, ¹⁵O, ¹⁸F. Un exemple de tels composés est le 2-[¹⁸F]fluoro-2-déoxy-D-glucose, communément appelé ¹⁸FDG.

La synthèse de ces composés radiopharmaceutiques de courte demi-vie est réalisée dans des appareils qui permettent la mise en contact des différents composés chimiques destinés à réaliser la synthèse, le chauffage de ceux-ci lors de la réaction et la purification des produits obtenus.

Afin d'éviter tout risque associé à la manipulation de substances radioactives, ces appareils sont placés dans un environnement blindé et contrôlé. Ces appareils sont généralement reliés à un automate qui commande les différentes opérations permettant de réaliser cette synthèse comprenant des étapes de réaction et de chauffage associées à des transferts de réactifs chimiques.

En raison de la courte demi-vie de ces radio-isotopes utilisés, il est nécessaire de réaliser régulièrement ce type de synthèse. Ceci multiplie les risques de contamination d'une synthèse à l'autre, alors que le produit final est destiné à être injecté à des patients.

En outre, un certain nombre de matériaux contenus dans les pièces de l'appareil de synthèse sont particulièrement sensibles à la radioactivité et/ou à la corrosion chimique. C'est le cas, par exemple, des vannes ou des tubes permettant les transferts.

Il faut donc régulièrement laver et stériliser toutes les pièces de l'appareil de synthèse, en particulier toute la tuyauterie et toutes les vannes qui assurent le transfert des réactifs, d'où la nécessité d'utiliser des pièces facilement stérilisables. Les utilisateurs doivent en outre assurer une maintenance approfondie, attentive et régulière de l'ensemble des pièces de l'appareil.

On connaît par le document US-A-5759513 un appareil et un procédé de préparation de composés radiochimiques dans lequel les réactifs nécessaires, à l'exclusion du réactif marqué au moyen d'un élément de courte demi-vie, sont dosés au préalable dans des conteneurs. On constitue alors un kit contenant lesdits conteneurs, les réacteurs, les colonnes et les tubes de transfert munis de vannes, nécessaires au procédé. Ce kit comprend un grand nombre d'éléments, y compris des vannes. Le coût de l'ensemble ne permet pas d'en faire un kit à usage unique.

On connaît par le document WO-A-93/11871 un appareil de préparation de composés radiochimiques, comportant un module à usage unique. Cet appareil est destiné à être raccordé à des conteneurs de réactifs, et comporte des moyens de dosage de ces réactifs, constitués d'injecteurs munis de pistons et mus par des moteurs pas à pas. Des moyens complexes, coûteux et imposant des manipulations à l'opérateur sont nécessaires pour relier les moteurs pas à pas aux tiges des injecteurs.

Le document US-A-5 415 843 décrit un appareil pour la fabrication de composés radiopharmaceutiques qui présente certaines caractéristiques communes avec celui décrit dans le document WO-A-93/11871. Cet appareil inclue un kit jetable à usage unique qui comprend une plaque et des récipients, mais pas les tubes de connexion entre les différents récipients.

Le document US-A-5 312 592 décrit lui aussi un appareil qui présente certaines caractéristiques communes avec celui décrit dans le document WO-A-93/11871. Ce document se rapporte plus particulièrement un kit jetable utilisable dans ledit appareil et comprenant une plaque pourvue d'ouvertures et d'embouts pour supporter des injecteurs de liquides et pour connecter des tuyaux de transfert, ainsi qu'un récipient d'hydrolyse.

L'article de Chaly T. et al., Appl.Radiat.Isot. vol.41, no.1,pp.29-34 (1990) dans Int. J. « Radiat. Appl. Instrum. Part A » (Pergamon Press) décrit un procédé pour produire du ¹⁸FDG en utilisant une unité de synthèse comprenant des éléments stériles jetables tels que des seringues, des aiguilles, des tubes, des adaptateurs, ainsi que des robinets d'arrêt.

### Buts de l'invention

La présente invention vise à fournir un dispositif pour la synthèse de produits radioactifs, en particulier de produits radiopharmaceutiques, à partir d'éléments radioactifs de courte demi-vie, qui soit facilement utilisable.

En particulier, la présente invention vise à fournir un dispositif qui minimise les risques de contamination d'une synthèse à l'autre.

La présente invention vise également à fournir un dispositif dont l'entretien et la maintenance sont facilités.

En particulier, la présente invention vise à fournir un dispositif qui est facilement lavable et facilement stérilisable.

### Résumé de l'invention

La présente invention se rapporte à un dispositif tel que défini dans la revendication 9 pour la synthèse de produits radiopharmaceutiques au départ de réactifs chimiques contenus dans des flacons, ledit dispositif comprenant plusieurs compartiments de réaction, des moyens de transfert entre lesdits flacons et lesdits compartiments de réaction ainsi que des moyens mécaniques agissant sur lesdits moyens de transfert et permettant de commander et de contrôler mécaniquement le transfert des réactifs chimiques, caractérisé en ce qu'il comporte :
- un module fixe comprenant au moins les moyens mécaniques,
- un module détachable et jetable tel que défini dans la revendication 1, se présentant essentiellement sous la forme d'une plaque support, sur lequel sont disposés les moyens de transfert entre lesdits flacons et lesdits compartiments de réaction, ledit module jetable et détachable ne comportant aucun moyen mécanique, et
- des moyens de solidarisation dudit module détachable et jetable audit module fixe.

Ledit module jetable comprend en outre lesdits flacons et de préférence, lesdits compartiments de réaction.

Les flacons sont des flacons prédosés.

La plaque support comprend des moyens de positionnement précis des moyens de transfert.

De préférence, les moyens de positionnement sont constitués par des rainures dans lesquelles viennent se placer les moyens de transfert.

De préférence, les moyens de transfert sont des tubes flexibles, les moyens mécaniques sont des pistons, les moyens de transfert, les moyens mécaniques et les moyens de positionnement étant aptes à coopérer afin de constituer des vannes .

De préférence, les moyens de solidarisation comprennent au moins une ouverture, des épaulements et des attaches.

De préférence, lesdits épaulements sont situés sur la plaque support, de préférence aux quatre coins de la plaque support, et sont capables de s'emboîter dans des attaches situées sur le module fixe.

De préférence, ladite ouverture est également située sur la plaque support, de préférence au centre de ladite plaque, et est capable de s'emboîter dans une attache située sur le module fixe.

De préférence, la nature de la plaque support (5) et celle des moyens de transfert sont telles qu'elles permettent un lavage dudit module jetable par autoclavage à la vapeur et/ou sa stérilisation.

De préférence, la plaque support est en ABS et les moyens de transfert sont en silicone.

De préférence, le dispositif comporte des moyens d'actionnement des moyens mécaniques du module fixe.

De préférence, les moyens d'actionnement sont constitués par un automate coopérant avec un ordinateur et faisant partie du module fixe.

La présente invention se rapporte également à un module jetable et détachable tel que défini dans la revendication 1 destiné à être associé à un module fixe par des moyens de solidarisation et se présentant essentiellement sous la forme d'une plaque support comprenant des moyens de positionnement des moyens de transfert de réactifs chimiques.

De préférence, ledit module comprend en outre des flacons et des compartiments de réaction.

De préférence, ledit module comprend également des colonnes.

De préférence, les moyens de positionnement sont constitués par des rainures capables de loger des moyens de transfert.

La présente invention se rapporte également à un procédé tel que défini dans la revendication 17 pour la synthèse de produits radiopharmaceutiques utilisant le dispositif selon la présente invention et comprenant les étapes suivantes :
- on dispose les moyens de transfert sur la plaque de support,
- on fixe les flacons contenant les réactifs chimiques à l'une des extrémités des tubes de transfert,
- on attache ladite plaque de support avec les moyens de transfert au module fixe à l'aide de moyens de solidarisation,
- on connecte les moyens de transfert aux différents éléments du module fixe,
- on commande via l'automate les différentes opérations nécessaires à la synthèse des produits pharmaceutiques, y compris l'actionnement des moyens mécaniques.

De préférence, avant de commander les différentes opérations nécessaires à la synthèse des produits pharmaceutiques, on effectue via l'automate un test pour vérifier l'étanchéité du dispositif.

Un autre objet de la présente invention est l'utilisation telle que définie dans la revendication 19 du dispositif selon la présente invention ou du module selon la présente invention pour la synthèse de composés radiopharmaceutiques contenant des radioéléments de courte demi-vie, de préférence le ¹¹C, le ¹³N, le ¹⁵O ou le ¹⁸F.

### Brève description des figures

La figure 1 présente une vue générale du module jetable fixé sur le module fixe du dispositif selon la présente invention.

La figure 2 représente une vue en perspective du module fixe dans le dispositif selon la présente invention.

La figure 3 représente une vue en perspective de la face avant du module jetable sans le module fixe dans le dispositif selon la présente invention.

La figure 4 représente une vue en perspective de la face arrière du module jetable sans le module fixe dans le dispositif selon la présente invention.

La figure 5 montre, selon une forme préférée d'exécution de l'invention, le mécanisme de fermeture des tubes souples appartenant au module jetable par des pistons appartenant au module fixe.

### Description détaillée d'une forme d'exécution préférée de l'invention

Il est à noter que les figures présentées ici se rapportent à un dispositif destiné à la synthèse du ¹⁸FDG. Ces figures ne sont données qu'à titre d'exemple pour illustrer l'invention mais qu'elles n'en limitent en rien la portée. L'homme de l'art pourra aisément adapter les différents éléments du dispositif en fonction du type de synthèse à réaliser.

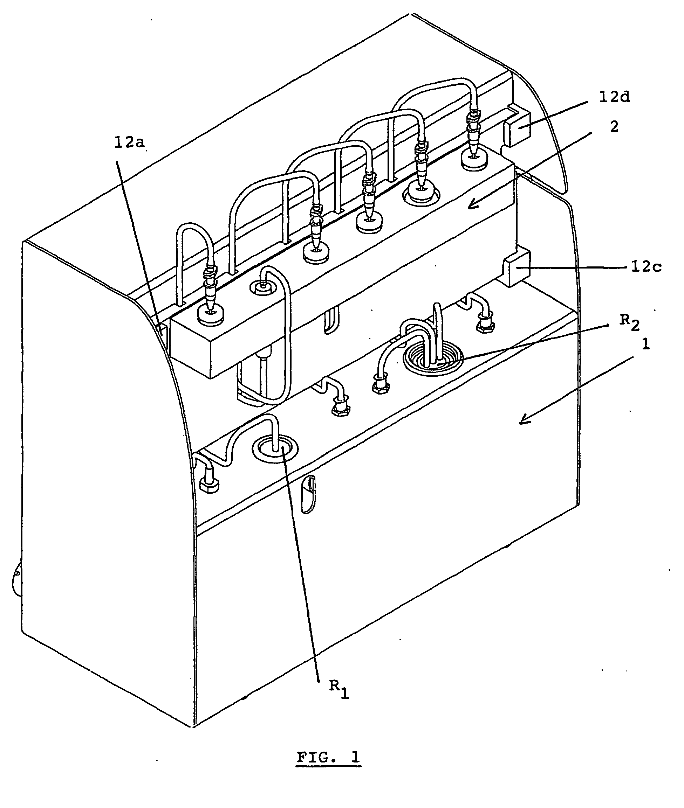

Comme illustré à la figure 1, le dispositif selon la présente invention comprend un module 1 fixe et un module 2 détachable et jetable à usage unique. Lorsque le dispositif fonctionne, le module 2 détachable et jetable est fixé sur le module 1 fixe.

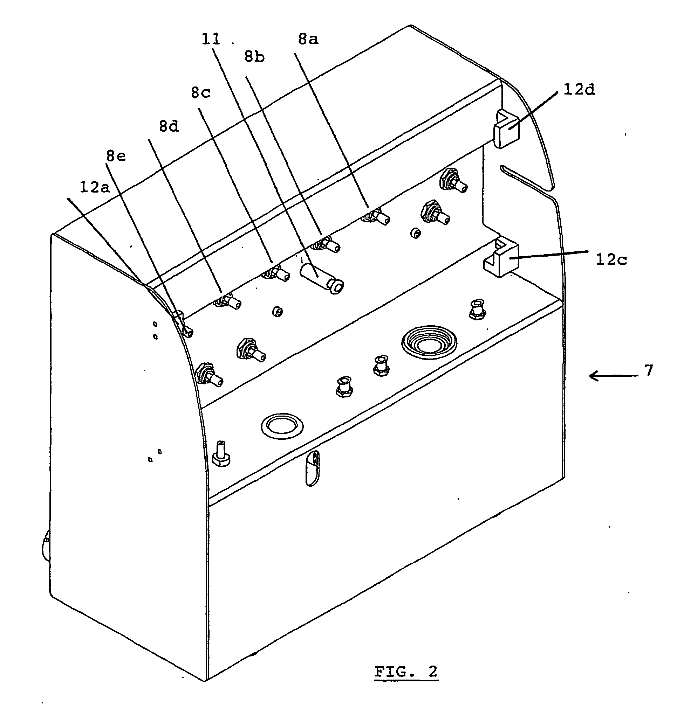

La figure 2 présente une vue détaillée du module fixe. Le module 1 fixe se présente ici sous la forme d'un coffret 7 sur lequel sont disposés des pistons 8a,8b,8c,8e,...

Le module 1 comprend également un automate piloté par un ordinateur, non représentés ici. L'automate commande toutes les opérations nécessaires à la mise en oeuvre de la synthèse des produits radiopharmaceutiques, en particulier le mouvement des pistons 8a,8b,8c,8d,8e,....

En outre, afin de pouvoir utiliser le dispositif, le module 1 comprend des moyens de solidarisation qui permettent de le fixer au module 2 jetable. Ces moyens de solidarisation peuvent prendre la forme d'attaches disposées selon une configuration précise. Dans le cas représenté à la figure 2, ces attaches sont de deux types. On distingue d'une part les quatre attaches 12a,12b,12c et 12d et l'attache centrale 11, ladite attache centrale 11 se présentant ici sous la forme d'un piton. La présence de l'attache centrale 11 a pour but de sécuriser la fixation du module 2 sur le module 1. Elle permet en outre. de contrecarrer la pression exercée par les pistons 8a,8b,8c,8d,8e,....

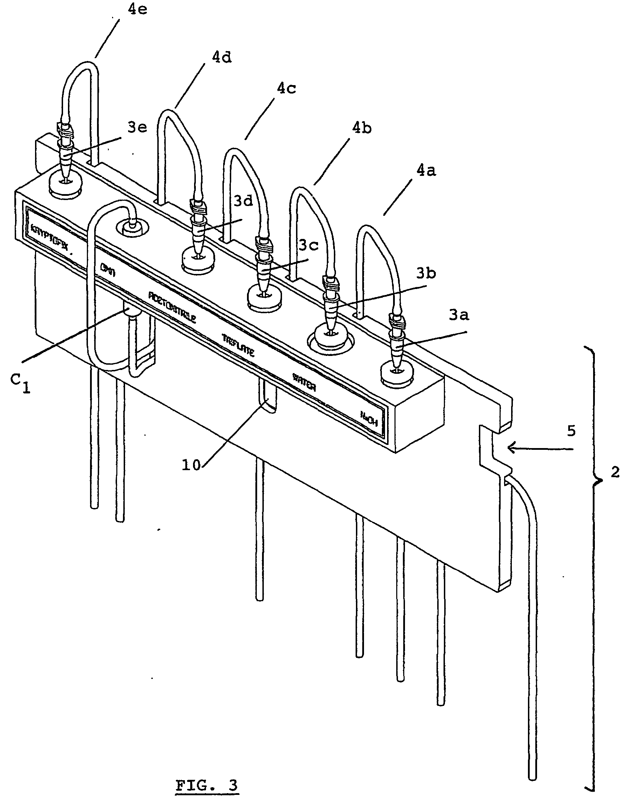

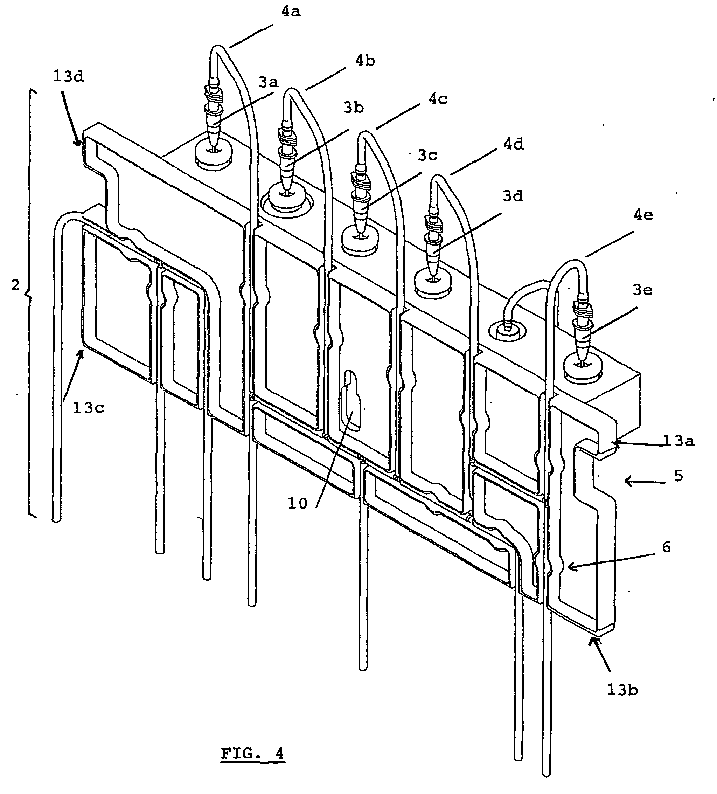

Les figures 3 et 4 présentent plus en détail les différents éléments du module jetable selon la présente invention. Le module 2 jetable comprend au moins:
- une plaque 5, dans laquelle est creusé un réseau de rainures 6 selon une configuration précise ;
- cinq flacons 3a,3b,3c,3d,3e contenant les réactifs chimiques prédosés, respectivement du Kryptofix, du K₂CO₃, de l'acétonitrile, du triflate, de l'eau et de la soude ;
- des tubes 4a,4b,4c,4d,4e,... pour faire circuler les produits chimiques à l'intérieur du dispositif de synthèse, ces tubes étant flexibles, dans le cas présent, et une de leurs extrémités étant connectée aux flacons 3a,3b,3c,3d,3e, respectivement,
- des colonnes, y compris des colonnes de sortie (non représentées). Le module 1 comprend ici au moins quatre colonnes, la colonne C₁ telle que représentée sur la figure 3 et les colonnes C₂, C₃, C₄, non représentées,
- des compartiments de réaction R₁ et R₂, tels que représentés à la figure 1.

Ladite plaque 5 a une double fonction. Une première fonction est que la plaque 5 sert de support aux tubes 4a,4b,4c,4d,4e quand on fixe ces tubes dans les rainures 6. La configuration de ces rainures 6 permet un positionnement très précis des tubes 4a,4b,4c,4d,4e les uns par rapport aux autres. En d'autres termes, les tubes 4a-4e, les pistons 8a-8e et les rainures 6 sont aptes à coopérer afin de constituer des vannes. Les pistons 8a-8e associés à la portion des tubes 4a-4e qui leur font face forment ainsi des vannes de type "pinch valves", c'est-à-dire des valves à piston.

Il est donc important que les tubes soient correctement positionnés afin de permettre à ces vannes de fonctionner correctement. En particulier, les pistons 8a-8e doivent pouvoir agir précisément sur lesdits tubes 4a-4e.

La deuxième fonction de la plaque 5 est qu'elle sert d'interface entre le module 1 fixe et le module 2 jetable. La plaque 5 est pourvue de moyens de solidarisation qui permettent de fixer ledit module 2 jetable sur ledit module 1 fixe. Ces moyens de solidarisation situés sur le module 2 jetable sont, de par leur forme, complémentaires des moyens de solidarisation situés sur le module 1 fixe.

Selon l'exemple d'exécution représenté aux figures 3 et 4, ces moyens de solidarisation prennent en d'une part la forme d'épaulements 13a,13b,13c,13d situés chacun à un coin de la plaque 5. Ces épaulements 13a,13b,13c,13d sont capables de s'emboîter dans les attaches respectivement 12a,12b,12c,12d. Les moyens de solidarisation prennent d'autre part la forme d'une ouverture 10 située au centre de la plaque 5 et dans laquelle peut s'emboîter l'attache centrale 11 du module 1 fixe. L'ouverture 10, de par sa position centrale au niveau de la plaque 5, permet d'assurer une solidarisation optimale du module 2 jetable au module 1 fixe lors de l'utilisation du dispositif.

Les moyens de solidarisation peuvent néanmoins prendre d'autres formes.

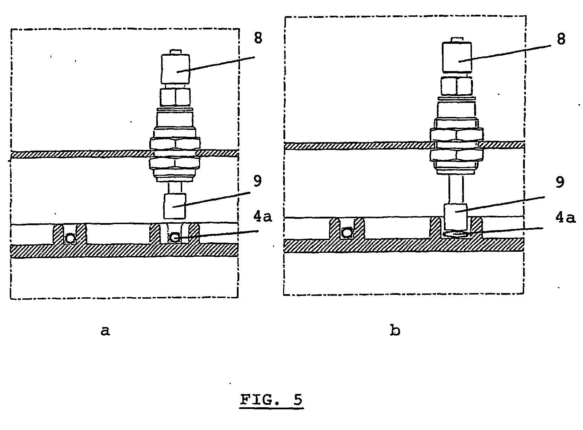

La figure 5 explique comment les pistons 8a-8e situés sur le module 1 fixe, en fonction du mouvement qu'il leur est commandé par l'automate, provoquent soit l'ouverture soit la fermeture des tubes 4a-4e du module 2 jetable. Cette figure montre, vue a, la tête 9 d'un de ces pistons 8a et une section d'un des tubes souples 4a en position ouverte, c'est-à-dire lorsque le tube 4a est ouvert. Dans cette position, la tête 9 du piston 8a est dégagée du tube 4a et les transferts de produits chimiques sont alors autorisés. Par contre, lorsque l'automate le commande, la tête 9 du piston 8a se déplace et vient exercer une pression sur la section du tube 4a, comme illustré à la vue b, et le tube 4a est alors fermé, ce qui empêche tout transfert de produits chimiques.

Par rapport aux dispositifs de l'état de la technique, la présente invention présente l'originalité d'être dépourvue de tout moyen mécanique, notamment de moyen mécanique pour ouvrir et fermer les tubes, ces moyens mécaniques étant situés sur le module 1 fixe.

De ce fait, le module 2 est facilement attachable et détachable. Il se fixe facilement sur le module 1 fixe. Un tel module 2 présente également l'avantage d'être peu coûteux, en particulier du fait de l'absence de moyens mécaniques dans ce module, et permet de ce fait un usage unique.

Selon une forme préférée d'exécution, la plaque 5 est en ABS et les tubes 4a, 4b, 4c, 4d, 4e, ... sont en silicone. L'avantage d'un tel choix au niveau des matériaux utilisés est que ces éléments sont capables de supporter les fortes pressions, en particulier les pressions qu'exercent les pistons, lors de l'utilisation du dispositif. Il permet également de limiter les déchets résultant d'une incinération de modules jetables utilisés.

Les différents éléments constituant ce module, pour autant que leur composition le permette, peuvent être facilement stérilisés, en particulier par les techniques conventionnelles telles que la stérilisation par irradiation gamma.

L'exemple décrit ci-dessous est un exemple d'utilisation du dispositif selon la présente invention pour la synthèse du ¹⁸FDG. Cet exemple n'est cependant donné qu'à titre d'illustration. D'autres utilisations sont possibles.

Pour la synthèse de ¹⁸FDG avec le dispositif selon la présente invention, on fait, dans un premier temps l'extraction de ¹⁸F⁻ du mélange H₂¹⁸O-H₂O-¹⁸F⁻. Pour cela, on transfère le mélange au moyen d'hélium gazeux jusqu'à la colonne C₁, contenant une résine échangeuse d'anions, telle que QMA Waters™. La colonne C₁ permet la récupération du ¹⁸F⁻, tandis que H₂¹⁸O-H₂O est transféré dans le compartiment de réaction R1.

Le tube 4e est ouvert pour transférer à l'aide d'une pompe à vide dans un compartiment de réaction ou réacteur R₂, 0.75 ml d'une solution de K₂CO₃/K2.2.2/H₂O/CH₃CN contenue dans le flacon 3e au travers de la colonne C1. On élue ainsi le ¹⁸F- et on le transfère dans le compartiment de réaction R₂.

On augmente la température du réacteur R₂ jusqu'à 90°C et on élimine H₂O en formant un azéotrope avec l'acétonitrile.

On ouvre ensuite le tube 4d et on transfère à l'aide d'une pompe à vide dans le compartiment de réaction R2 0.5 ml d'acétonitrile contenu dans le flacon 3d.

On élimine les traces de H₂O en formant un azéotrope avec de l'acétonitrile. On poursuit l'évaporation pour terminer le séchage.

On ouvre le tube 4c de façon à transférer à l'aide d'une pompe à vide une solution de 13 mg de 1,3,4,6-tétra-O-acétyl-2-O-trifluorométhane-sulfonyl-β-D-mannopyranose dans 1 ml d'acétonitrile contenu dans le flacon 3c dans le compartiment R₂. La température dudit compartiment R₂ est amenée à 85°C.

On évapore ensuite l'acétonitrile sous vide tout en maintenant la température dans le réacteur R₂ entre 90 et 85°C.

On ouvre ensuite le tube 4b de façon à transférer à l'aide d'une pompe à vide 1 ml d'H₂O contenu dans le flacon 3b. Le réacteur R₂ est alors refroidi.

On ouvre ensuite le tube 4a de façon à transférer à l'aide d'une pompe à vide 1 ml d'une solution de NaOH 0.5 N contenue dans le flacon 3a dans le réacteur R₂.

On transfère à l'aide d'azote à une pression de 400 mbar le contenu du réacteur R₂ au travers des colonnes de purification C₂, C₃, C₄, non représentées, disposées en série à la sortie du circuit.

On ouvre une seconde fois le tube 4b pour transférer à l'aide d'une pompe à vide 4 ml d'H₂O du flacon 3b dans le réacteur R₂.

On transfère une deuxième fois à l'aide d'azote à une pression de 400 mbar le contenu du réacteur R₂ au travers des colonnes de purification C₂, C₃, C₄.

On notera enfin que le dispositif selon la présente invention offre l'avantage d'être particulièrement compact, réduisant ainsi le volume de la chambre blindée dans laquelle il est utilisé, et par là, de la quantité de plomb.

## Revendications

1. Module (2) jetable pour utilisation dans un dispositif de synthèse de produits radiopharmaceutiques au départ de réactifs chimiques, ledit module (2) jetable se présentant essentiellement sous la forme d'une plaque support (5) et comprenant en outre:
- des moyens de transfert (4a,4b,4c,4d,4e, ...) desdits réactifs chimiques ;
- des flacons (3a, 3b, 3c, 3d, 3e, ...) contenant les réactifs chimiques;
ladite plaque support (5) étant pourvue de moyens de positionnement pour positionner lesdits moyens de transfert, et ledit module jetable (2) étant **caractérisé en ce que** lesdits flacons contenant lesdits réactifs chimiques sont des flacons prédosés.

2. Module jetable (2) selon la revendication 1, **caractérisé en ce qu'**il est dépourvu de moyen mécanique.

3. Module jetable (2), selon l'une des revendications précédentes, **caractérisé en ce que** lesdits moyens de positionnement sont constitués par des rainures (6) dans lesquelles viennent se placer les moyens de transfert (4a,4b,4c,4d,4e,...).

4. Module jetable (2) selon l'une des revendications précédentes, **caractérisé en ce que** les moyens de transfert sont des tubes (4a, 4b, 4c, 4d, 4e, ...) flexibles.

5. Module jetable (2) selon l'une des revendications précédentes, **caractérisé en ce qu'**il comprend en outre des compartiments de réaction (R₁,R₂).

6. Module jetable (2) selon l'une des revendications précédentes, **caractérisé en ce que** la nature de la plaque support (5) et celle des moyens de transfert (4a, 4b, 4c, 4d, 4e) sont telles qu'elles permettent un lavage dudit module (2) jetable par autoclavage à la vapeur et/ou sa stérilisation.

7. Module jetable (2) selon la revendication 5, **caractérisé en ce que** la plaque support (5) est en ABS.

8. Module jetable (2) selon la revendication 5 ou 6, **caractérisé en ce que** les moyens de transfert (4a,4b,4c,4d,4e, ...) sont en silicone.

9. Dispositif pour la synthèse de produits radiopharmaceutiques au départ de réactifs chimiques contenus dans des flacons (3a,3b,3c,3d,3e), ledit dispositif comprenant un module jetable (2) selon l'une des revendications précédentes, ainsi qu'un module fixe (1) et des moyens de solidarisation (10, 11, 12a-12d, 13a-13d) dudit module (2) jetable audit module (1) fixe.

10. Dispositif selon la revendication 9, **caractérisé en ce que** ledit module fixe (1) comprend au moins des moyens mécaniques (8a,8b,8c,8d,8e,...) agissant sur les moyens de transfert (4a,4b,4c,4d,4e,...) dudit module jetable (2) et permettant de commander et de contrôler mécaniquement le transfert des réactifs chimiques depuis lesdits flacons (3a,3b,3c,3d,3e) dudit module jetable (2).

11. Dispositif selon l'une des revendications 9 ou 10, **caractérisé en ce que** les moyens mécaniques (8a, 8b, 8c, 8d, 8e, ...) sont des pistons, lesdits moyens mécaniques, les moyens de transfert (4a, 4b, 4c, 4d, 4e, ...) et les moyens de positionnement (6) étant aptes à coopérer afin de constituer des vannes

12. Dispositif selon l'une quelconque des revendications 9 à 11, **caractérisé en ce que** les moyens de solidarisation comprennent au moins une ouverture (10), des épaulements (13a-13d) et des attaches (11, 12a-12d).

13. Dispositif selon la revendication 12, **caractérisé en ce que** lesdits épaulements (13a-13d) sont situés sur la plaque support (5), de préférence aux quatre coins de la plaque support (5), et sont capables de s'emboîter dans des attaches (12a-12d) situées sur le module fixe (1).

14. Dispositif selon la revendication 12, **caractérisé en ce que** ladite ouverture (10) est également située sur la plaque support (5), de préférence au centre de ladite plaque (5), et est capable de s'emboîter dans une attache (11) située sur le module fixe (1) .

15. Dispositif selon l'une quelconque des revendications précédentes, **caractérisé en ce qu'**il comporte des moyens d'actionnement des moyens mécaniques du module fixe (1).

16. Dispositif selon l'une quelconque des revendications précédentes, **caractérisé en ce que** les moyens d'actionnement sont constitués par un automate coopérant avec un ordinateur et faisant partie du module fixe (1) .

17. Procédé pour la synthèse de produits radiopharmaceutiques utilisant le module jetable (2) selon l'une des revendications 1 à 8 ou le dispositif selon l'une des revendications 9 à 16, **caractérisé en ce qu'**il comprend les étapes suivantes :
- au niveau du module jetable (2), on dispose les moyens de transfert (4a,4b,4c,4d,4e) sur la plaque (5) de support,
- on fixe, au niveau du module jetable (2), les flacons (3a-3e) contenant les réactifs chimiques à l'une des extrémités des moyens de transfert (4a,4b,4c,4d,4e),
- on attache, à l'aide de moyens de solidarisation (10, 11, 12a-12d, 13a-13d), le module jetable (2), c'est-à-dire ladite plaque (5) de support avec les moyens de transfert, au module fixe (1),
- on connecte les moyens de transfert (4a,4b,4c,4d,4e) du module jetable (2) aux différents éléments du module fixe (1),
- on commande via un automate les différentes opérations nécessaires à la synthèse des produits pharmaceutiques, y compris l'actionnement des moyens mécaniques (8a-8e) du module fixe (1).

18. Procédé selon la revendication 17, **caractérisé en ce qu'**avant de commander les différentes opérations nécessaires à la synthèse des produits pharmaceutiques, on effectue via l'automate un test pour vérifier l'étanchéité du dispositif.

19. Utilisation du module jetable (2) selon l'une des revendications 1 à 8 ou du dispositif selon l'une quelconque des revendications 9 à 16 pour la synthèse de composés radiopharmaceutiques contenant des radioéléments de courte demi-vie, de préférence le ¹¹C, le ¹³N, le ¹⁵O ou le ¹⁸F.

## Patentansprüche

1. Wegwerfmodul (2) zur Verwendung in einer Vorrichtung zur Synthese radiopharmazeutischer Produkte aus chemischen Reagenzien, wobei das Wegwerfmodul (2) im wesentlichen aus einer Trägerplatte (5) besteht und außerdem:
- Mittel (4a,4b,4c,4d,4e,...) zum Übertragen der chemischen Reagenzien;
- Flaschen (3a,3b,3c,3d,3e,...), die die chemischen. Reagenzien enthalten umfasst,
wobei die besagte Trägerplatte (5) mit Positionierungsmitteln zum Positionieren der besagten Übertragungsmittel versehen ist, und das besagte Wegwerfmodul (2) **dadurch gekennzeichnet ist, dass** die besagten Flaschen, die die chemischen Reagenzien enthalten, vordosierte Flaschen sind.

2. Wegwerfmodul (2) nach Anspruch 1, **dadurch gekennzeichnet, dass** es frei von mechanischen Mitteln ist.

3. Wegwerfmodul (2) nach einem der vorhergehenden Ansprüche, **dadurch gekennzeichnet, dass** die besagten Positionierungsmittel aus Rillen (6) bestehen, in die sich die Übertragungsmittel (4a,4b,4c,4d,4e,...) setzen.

4. Wegwerfmodul (2) nach einem der vorhergehenden Ansprüche, **dadurch gekennzeichnet, dass** die Übertragungsmittel (4a, 4b, 4c, 4d, 4e,...) flexible Röhren sind.

5. Wegwerfmodul (2) nach einem der vorhergehenden Ansprüche, **dadurch gekennzeichnet, dass** es außerdem Reaktionsräume (R₁, R₂) umfasst.

6. Wegwerfmodul (2) nach einem der vorhergehenden Ansprüche, **dadurch gekennzeichnet, dass** die Trägerplatte (5) und die Übertragungsmittel (4a,4b,4c,4d,4e) so beschaffen sind, dass sie ein Waschen des besagten Wegwerfmoduls (2) durch Autoklavbeandlung mittels Dampf und/oder seine Sterilisation ermöglichen.

7. Wegwerfmodul (2) nach Anspruch 5, **dadurch gekennzeichnet, dass** die Trägerplatte (5) aus ABS ist.

8. Wegwerfmodul nach Anspruch 5 oder 6, **dadurch gekennzeichnet, dass** die Übertragungsmittel (4a,4b,4c,4d,4e,...) aus Silikon sind.

9. Vorrichtung zur Synthese radiopharmazeutischer Produkte aus chemischen Reagenzien, die in Flaschen (3a,3b,3c,3d,3e) enthalten sind, welche Vorrichtung ein Wegwerfmodul (2) nach einem der vorhergehenden Ansprüche sowie ein festes Modul (1) und Mittel (10, 11, 12a-12d, 13a-13-d) zum Verbinden des besagten Wegwerfmoduls (2) an dem besagten festen Modul (1) umfasst.

10. Vorrichtung nach Anspruch 9, **dadurch gekennzeichnet, dass** das besagte feste Modul (1) mindestens mechanische Mittel (8a,8b,8c,8d,8e...) umfasst, die auf die Übertragungsmittel (4a,4b,4c,4d,4e,...) des besagten Wegwerfmoduls (2) einwirken und es ermöglichen, die Übertragung chemischer Reagenzien aus den besagten Flaschen (3a,3b,3c,3d,3e) des besagten Wegwerfmoduls (2) mechanisch zu steuern.

11. Vorrichtung nach einem der Ansprüche 9 oder 10, **dadurch gekennzeichnet, dass** die mechanischen Mittel (8a,8b,8c,8d,8e,...) Kolben sind, wobei die besagten mechanischen Mittel, die Übertragungsmittel (4a,4b,4c,4d,4e,...) und die Positionierungsmittel (6) in der Lage sind, zusammenzuarbeiten, um Ventile zu bilden.

12. Vorrichtung nach irgendeinem der Ansprüche 9 bis 11, **dadurch gekennzeichnet, dass** die Verbindungsmittel mindestens eine Öffnung (10), Absätze (13a-13d) und Befestigungen (11, 12a-12d) umfassen.

13. Vorrichtung nach Anspruch 12, **dadurch gekennzeichnet, dass** die besagten Absätze (13a-13d) sich auf der Trägerplatte (5) befinden, vorzugsweise an den vier Ecken der Trägerplatte (5), und in Befestigungen (12a-12d) passen können, die sich auf dem festen Modul (1) befinden.

14. Vorrichtung nach Anspruch 12, **dadurch gekennzeichnet, dass** die besagte Öffnung (10) sich ebenfalls auf der Trägerplatte (5) befindet, vorzugsweise in der Mitte der besagten Platte (5), und in eine Befestigung (11) passen kann, die sich auf dem festen Modul (1) befindet.

15. Vorrichtung nach irgendeinem der vorhergehenden Ansprüche, **dadurch gekennzeichnet, dass** sie Mittel zum Betätigen der mechanischen Mittel des festen Moduls (1) umfasst.

16. Vorrichtung nach irgendeinem der vorhergehenden Ansprüche, **dadurch gekennzeichnet, dass** die Betätigungsmittel aus einem Automaten bestehen, der mit einem Computer zusammenarbeitet und Teil des festen Moduls (1) ist.

17. Verfahren zur Synthese radiopharmazeutischer Produkte unter Verwendung des Wegwerfmoduls (2) nach einem der Ansprüche 1 bis 8 oder der Vorrichtung nach einem der Ansprüche 9 bis 16, **dadurch gekennzeichnet, dass** sie folgende Schritte umfasst:
- auf dem Wegwerfmodul (2) ordnet man die Übertragungsmittel (4a,4b,4c,4d,4e) auf der Trägerplatte (5) an,
- auf dem Wegwerfmodul (2) befestigt man die Flaschen (3a-3e), die die chemischen Reagenzien enthalten, an einem der Enden der Übertragungsmittel (4a,4b,4c,4d,4e),
- mit Hilfe von Verbindungsmitteln (10, 11, 12a-12d, 13a-13d) befestigt man das Wegwerfmodul (2), das heißt, die besagte Trägerplatte (5) mit den Übertragungsmitteln, an dem festen Modul (1),
- man verbindet die Übertragungsmittel (4a,4b,4c,4d,4e) des Wegwerfmoduls (2) mit den verschiedenen Elementen des festen Moduls (1);
- man steuert über einen Automaten die verschiedenen Operationen, die zur Synthese pharmazeutischer Produkte erforderlich sind, einschließlich die Betätigung mechanischer Mittel (8a-8e) des festen Moduls (1).

18. Verfahren nach Anspruch 17, **dadurch gekennzeichnet, dass** vor dem Steuern der verschiedenen Operationen, die zur Synthese pharmazeutischer Produkte erforderlich sind, man über den Automaten einen Test durchführt, um die Dichtheit der Vorrichtung zu überprüfen.

19. Verwendung des Wegwerfmoduls (2) nach einem der Ansprüche 1 bis 8 oder der Vorrichtung nach einem der Ansprüche 9 bis 16 zur Synthese radiopharmazeutischer Verbindungen, die Radioelemente mit kurzer Halbwertzeit enthalten, vorzugsweise ¹¹C, ¹³N, ¹⁵O oder ¹⁸F.

## Claims

1. Disposable module (2) for use in a device for synthesis of radiopharmaceutical products from chemical reagents, the said disposable module (2) being essentially in the form of a support plate (5) and furthermore comprising:
- means (4a,4b,4c,4d,4e,...) for transferring the said chemical reagents;
- bottles (3a,3b,3c,3d,3e,...) containing the chemical reagents;
the said support plate (5) being provided with positioning means for positioning the said transfer means, and the said disposable module (2) being **characterised in that** the said bottles containing the said chemical reagents are pre-metered bottles.

2. Disposable module (2) according to claim 1, **characterised in that** it does not have any mechanical means.

3. Disposable module (2) according to one of the preceding claims, **characterised in that** the said positioning means are constituted by grooves (6) in which the transfer means (4a,4b,4c,4d,4e,...) are placed.

4. Disposable module (2) according to one of the preceding claims, **characterised in that** the transfer means are flexible tubes (4a,4b,4c,4d,4e,...).

5. Disposable module (2) according to one of the preceding claims, **characterised in that** it furthermore comprises reaction compartments (R₁,R₂).

6. Disposable module (2) according to one of the preceding claims, **characterised in that** the nature of the support plate (5) and that of the transfer means (4a,4b,4c,4d,4e) are such that they allow washing of the said disposable module (2) by steam autoclaving and/or its sterilisation.

7. Disposable module (2) according to claim 5, **characterised in that** the support plate (5) is made of ABS.

8. Disposable module (2) according to claim 5 or 6, **characterised in that** the transfer means (4a,4b,4c,4d,4e,...) are made of silicone.

9. Device for synthesis of radiopharmaceutical products from chemical reagents contained in bottles (3a,3b,3c,3d,3e), the said device comprising a disposable module (2) according to one of the preceding claims as well as a fixed module (1) and means (10, 11, 12a-12d, 13a-13d) of securing the said disposable module (2) to the said fixed module (1).

10. Device according to claim 9, **characterised in that** the said fixed module (1) comprises at least mechanical means (8a,8b,8c,8d,8e,...) acting on the transfer means (4a,4b,4c,4d,4e,...) of the said disposable module (2) and enabling to monitor and control mechanically the transfer of the chemical reagents from the said bottles (3a,3b,3c,3d,3e) of the said disposable module (2).

11. Device according to one of claims 9 or 10, **characterised in that** the mechanical means (8a,8b,8c,8d,8e,...) are pistons, the said mechanical means, the transfer means (4a,4b,4c,4d,4e,...) and the positioning means (6) being capable of co-operating so as to form valves.

12. Device according to any of claims 9 to 11, **characterised in that** the securing means comprise at least one opening (10), shoulders (13a-13d) and fasteners (11, 12a-12d) .

13. Device according to claim 12, **characterised in that** the said shoulders (13a-13d) are located on the support plate (5), preferably at the four corners of the support plate (5), and are capable of fitting into fasteners (12a-12d) located on the fixed module (1).

14. Device according to claim 12, **characterised in that** the said opening (10) is likewise located on the support plate (5), preferably at the centre of the said plate (5), and is capable of fitting into a fastener (11) located on the fixed module (1).

15. Device according to any of the preceding claims, **characterised in that** it includes means of actuating mechanical means of the fixed module (1).

16. Device according to any of the preceding claims, **characterised in that** the actuating means are constituted by an automaton co-operating with a computer and forming part of the fixed module (1).

17. Method for synthesising radiopharmaceutical products using the disposable module (2) according to one of claims 1 to 8 or the device according to one of claims 9 to 16, **characterised in that** it comprises the following stages:
- at the disposable module (2), the transfer means (4a,4b,4c,4d,4e) are arranged on the support plate (5),
- at the disposable module (2), the bottles (3a-3e) containing the chemical reagents are fixed to one of the ends of the transfer means (4a,4b,4c,4d,4e),
- the disposable module (2), that is to say the said support plate (5) with the transfer means, is attached to the fixed module (1) with the aid of securing means (10, 11, 12a-12d, 13a-13d),
- the transfer means (4a,4b,4c,4d,4e) of the disposable module (2) are connected to the various elements of the fixed module (1),
- the various operations required for synthesis of the pharmaceutical products, including the actuation of the mechanical means (8a-8e) of the fixed module are commanded via an automaton.

18. Method according to claim 17, **characterised in that** before commanding the various operations required for synthesis of the pharmaceutical products, a test to verify the tightness of the device is carried out via the automaton.

19. Use of the disposable module (2) according to one of claims 1 to 8 or of the device according to any of claims 9 to 16 for synthesising radiopharmaceutical compounds containing radio-elements with a short half-life, preferably ¹¹C, ¹³N, ¹⁵O or ¹⁸F.
